Europäisches Patentamt

European Patent Office

Office européen des brevets

(11) Veröffentlichungsnummer: **0 306 843**
**A2**

## (12) EUROPÄISCHE PATENTANMELDUNG

(21) Anmeldenummer: 88114308.5

(22) Anmeldetag: 02.09.88

(51) Int. Cl.⁴: **A61K 7/08 , B01F 17/00**

(30) Priorität: 09.09.87 DE 3730179

(43) Veröffentlichungstag der Anmeldung:
15.03.89 Patentblatt 89/11

(84) Benannte Vertragsstaaten:
**AT BE CH DE ES FR GB GR IT LI NL SE**

(71) Anmelder: **Henkel Kommanditgesellschaft auf Aktien**
**Postfach 1100 Henkelstrasse 67**
**D-4000 Düsseldorf-Holthausen(DE)**

(72) Erfinder: **Tesmann, Holger, Dr.**
**Vennstrasse 61**
**D-4000 Düsseldorf 12(DE)**
Erfinder: **Hensen, Hermann, Dr.**
**Rathmacherweg 13**
**D-5657 Haan(DE)**
Erfinder: **Hochschon, Wolfgang**
**Ellerstrasse 46**
**D-4010 Hilden(DE)**
Erfinder: **Ploog, Uwe, Dr.**
**Haydnweg 6**
**D-5657 Haan(DE)**

(54) **Verdickte wässrige Tensidlösungen, insbesondere für deren Einsatz auf dem Gebiet kosmetischer Präparate.**

(57) Polyethoxylate ein- und/oder mehrfach ungesättigter Fettalkohole mit 12 - 18 C Atomen eines mittleren Ethoxylierungsgrades von 2 bis 5, die endständig auch 1 - 2 Propylenoxidgruppen anstelle der entsprechenden Zahl zn Ethylenoxidgruppen ankondensiert enthalten können, eignen sich, gegebenenfalls zusammen mit gelösten anorganischen Elektrolytsalzen, als N-freie Verdickungsmittel für wäßrige Tensidlösungen. Die Produkte können auch in Abmischung mit Polyethoxylaten gesättigter Fettalkohole verwendet werden.

EP 0 306 843 A2

## Verdickte wäßrige Tensidlösungen, insbesondere für deren Einsatz auf dem Gebiet kosmetischer Präparate

Die Erfindung betrifft das Gebiet der Verdickung von wäßrigen Tensidlösungen, insbesondere für deren Einsatz auf dem Gebiet kosmetischer Präparate, die in den Bereich der Körperreinigungsmittel fallen und beispielsweise als Haarshampoo, Schaumbäder, Duschbäder, Handwaschpasten und dergleichen zum Einsatz kommen.

Wirkstoffgemische dieser Art enthalten als hauptsächlichen Tensidanteil meist Aniontenside, beispielsweise Alkylethersulfate. Zur Stabilisierung der klaren oder dispersen Systeme und zu ihrer besseren Handhabung werden üblicherweise Verdickungsmittel zugesetzt. Als Mittel zur Erhöhung der Viskosität aniontensidhaltiger Formulierungen sind eine Vielzahl von Verbindungen bekannt. Die einfachste und billigste Lösung zur Regulierung der Viskosität aniontensidhaltiger Lösungen besteht darin, daß man Kochsalz oder andere anorganische wasserlösliche Elektrolytsalze zusetzt. Neben oder anstelle dieser anorganischen Komponenten ist der Zusatz löslicher organischer Verbindungen mit Verdikkungswirkung üblich. So ist es bekannt, daß Fettsäurealkanolamide, z. B. Kokosfettsäuremonoethanolamid, Laurinsäuremonoethanolamid, Ölsäurediethanolamid und Kokosfettsäurediethanolamid auf viele Tensidsysteme eine verdickende und die Schaumstabilität unter Fettbelastung erhöhende Wirkung haben. Weiterhin ist bekannt, daß z. B. Polyethylenglycoldifettsäureester und viele wasserlösliche Polymere auch eine verdickende Wirkung auf wäßrige Tensidlösungen haben.

Die genannten Verdickungsmittel zeigen jedoch viele Nachteile. So weisen die mit Polyethylenglycolfettsäurediester verdickten Lösungen oft eine unzureichende Viskositätsstabilität bei Lagerung auf, wasserlösliche Polymere sind oft schwer auflösbar und bewirken ein unerwünschtes, schleimiges Fließverhalten mit der Neigung zum "Fädenziehen", was in kosmetischen Zubereitungen sehr unerwünscht ist. Fettsäurealkanolamide sind als Abkömmlinge von z. B. Diethanolamin in kosmetischen Zubereitungen zunehmend unerwünscht, da ein geringer Gehalt an freiem Alkanolamin als Nebenprodukt nicht restlos ausgeschlossen werden kann und Anlaß zur Nitrosaminbildung sein kann.

Aufgabe der Erfindung ist es, neue Verdickungsmittel für wäßrige Tensidzubereitungen zur Verfügung zu stellen, welche die vorgenannten Nachteile nicht besitzen und auch keine Derivate von Aminen oder Alkanolaminen sind.

Es wurde gefunden, daß sich die Viskosität wäßriger Zubereitungen von insbesondere ionischen Tensiden durch ausgewählte Polyethoxylate ein- und/oder mehrfach ungesättigter Fettalkohole wirkungsvoll erhöhen läßt, wobei die Verdickungswirkung der erfindungsgemäß besonders bevorzugten ungesättigten Fettalkohol-Polyethoxylate die entsprechende Wirkung vorbekannter Komponenten deutlich übertreffen kann.

Gegenstand der Erfindung ist dementsprechend in einer ersten Ausführungsform die Verwendung von Polyethoxylaten ein- und/oder mehrfach ungesättigter Fettalkohole mit 12 bis 22 C-Atomen, eines mittleren Ethoxylierungsgrades von etwa 2 bis 5 als N-freies Verdickungsmittel für wäßrige Tensidlösungen, die auch gelöste anorganische Elektrolytsalze mit Verdickungswirkung enthalten können. In den Rahmen der Erfindung fällt der Einsatz von Polyethoxylaten der genannten Art, die endständig eine oder maximal zwei Propylenoxidgruppen anstelle der entsprechenden Zahl an Ethylenoxidgruppen ankondensiert enthalten.

Besonders geeignet sind entsprechende ungesättigte Alkohol-Polyethoxylate eines mittleren Ethoxylierungsgrades im Bereich von etwa 2,5 bis 4,5 und insbesondere solche des Bereiches von etwa 3 bis 4. Optimale Verdickungswirkungen werden insbesondere im zuletzt genannten vergleichsweise engen Bereich und hier bevorzugt bei Werten um etwa 3,5 erzielt.

Die erfindungsgemäß zum Einsatz kommenden Polyethoxylate der ein- und/oder mehrfach ungesättigten Fettalkohole werden in einer bevorzugten Ausführungsform in Abmischung mit Polyethoxlaten gesättigter Fettalkohole verwendet. Es kann dabei insbesondere zweckmäßig sein, auch diese gesättigten Komponenten in den genannten Zahlenbereichen für die Kettenlänge der Alkoholkomponente und des Polyethoxylierungsgrades auszuwählen bzw. einzusetzen. Bevorzugte Fettalkohol-Polyethoxylatgemische enthalten überwiegende Anteile der Kohlenstoffkettenlänge $C_{16-20}$ und insbesondere $C_{16-18}$. Auch diese Zahlenwerte gelten sowohl für die ungesättigten Fettalkoholderivate wie für die gegebenenfalls mitverwendeten gesättigten entsprechenden Fettalkohol-Polyethoxylate. Auch die Möglichkeit der endständigen Propoxylierung gilt für die gesättigten Komponenten sinngemäß.

Fettalkoholethoxylate auf Basis ungesättigter Fettalkohole mit einer C-Kettenlänge von $C_{16}$-$C_{22}$ und Jodzahlen von 45 bis 170 bei einem Ethoxylierungsgrad von etwa 2 bis 5 sowie gewünschtenfalls mitverwendete gesättigte Fettalkoholethoxylate mit einer C-Kettenlänge von $C_{12}$-$C_{18}$ und einem Ethoxylierungsgrad von etwa 2 bis 5 stellen brauchbare Wirkstoffe bzw. Wirkstoffgemische im Sinne der Erfindung dar.

Die ungesättigten Fettalkohol-Polyethoxylate enthalten in den ungesättigten Fettalkoholresten üblicherweise 1 bis 3 ethylenische Doppelbindungen. Die entsprechenden einfach ethylenisch ungesättigten Komponenten stellen innerhalb der Gruppe der ungesättigten Fettalkohole in der Regel den Hauptteil dar, insbesondere wenn Einsatzmaterialien natürlichen Ursprungs verwendet werden, wie es im nachfolgenden noch geschildert wird.

Bevorzugt werden Gemische von gesättigten und ungesättigten Fettalkohol-Polyethoxylaten verwendet, die wenigstens etwa zur Hälfte bevorzugt zu etwa 55 bis 90 Gew.-% ungesättigte Fettalkohol-Polyethoxylate enthalten. Ungesättigte Fettalkohole bzw. ihre Abmischungen mit gesättigten Fettalkoholen können in an sich bekannter Weise aus Fetten, Fettsäureestern bzw. Fettsäuren natürlichen Ursprungs erhalten werden, wobei durch Auswahl geeigneter Katalysatoren sichergestellt wird, daß bei der reduzierenden Umwandlung der Carbonsäuregruppierung zur Alkoholgruppe in den Kohlenstoffketten vorliegende ethylenische Doppelbindungen nicht oder nicht wesentlich hydriert werden. Geeignete Fettalkoholgemische natürlichen Ursprungs mit beträchtlichem Gehalt an ungesättigten Komponenten sind beispielsweise die von der Anmelderin unter den Handelsnamen HD-Ocenol 50/55 und HD-Ocenol 80/85 vertriebenen Produkte etwa der nachfolgenden Zusammensetzung.

| C-Kette | HD-Ocenol 50/55 Gew.-% Spezifikation (typ. Gehalt) | | HD-Ocenol 80/85 Gew.-% Spezifikation (typ. Gehalt) | |
|---|---|---|---|---|
| C 12 | 0 - 2 | (ca. 1) | 0 - 2 | (ca. 1) |
| C 14 | 2 - 7 | (ca. 5) | 2 - 7 | (ca. 5) |
| C 16 | 25 - 35 | (ca. 30) | 8 - 18 | (ca. 13) |
| C 18 | | (ca. 20) | | |
| C 18' | 55 - 75 | (ca. 41) | 70 - 83 | (ca. 71) |
| C 18'' | | (ca. 1) | | (ca. 4) |
| C 18''' | | (ca. 1) | | (ca. 2) |
| C 20 | 0 - 2 | (ca. 1) | 0 - 3 | (ca. 2) |

Die C-Kettenverteilung eines Verdickungsmittels auf Basis eines aus Rüböl gewonnenen ungesättigten Fettalkohols ("Rübocenol") entspricht etwa der folgenden Zusammensetzung:

C-Kette Gew.-%

C 16 ca. 7,0
C 16' ca. 2,0 (Palmitoleylalkohol)
C 18 ca. 3,0
C 18' ca. 70,0 (Oleylalkohol)
C 18'' ca. 8,0 (Linoleylalkohol)
C 18''' ca. 7,0 (Linolenylalkohol)
C 20 ca. 3,0

In diesen Darstellungen ist die jeweilige Anzahl der vorliegenden ethylenischen Doppelbindungen durch die Anzahl der hochgesetzten '-Zeichen dargestellt.

In einer Ausführungsform betrifft die Erfindung verdickte wäßrige Zubereitungen ionischer Tenside, insbesondere für den Einsatz auf dem Gebiet kosmetischer Präparate enthaltend etwa 5 bis 20 Gew.-% ionischer Tenside zusammen mit etwa 1 bis 5 Gew.-% wasserlöslicher Verdickungsmittel und 0 bis 10

3

Gew.-% wasserlöslicher anorganischer Elektrolytsalze sowie gewünschtenfalls übliche Zusatzstoffe kosmetischer Zubereitungen in untergeordneten Mengen, wobei das Kennzeichen für die erfindungsgemäße Lehre darin liegt, daß diese Zubereitungen als N-freies Verdickungsmittel Polyethylenoxidverbindungen der geschilderten Art von ein- und/oder mehrfach ungesättigten Fettalkoholen mit 12 bis 22 C-Atomen eines mittleren Polyethylenoxid-Grades von etwa 2 bis 5, bevorzugt von etwa 3 bis 4 - gegebenenfalls in Abmischung mit Polyethylenoxidverbindungen gesättigter Fettalkohole - enthalten.

Als wasserlösliche ionische Tenside können anionische, zwitterionische und kationische Tenside enthalten sein. Geeignete ionische Tenside zeichnen sich durch eine lipophile, bevorzugt lineare Alkyl- oder Alkenylgruppe mit 8 - 18 C-Atomen und eine, bevorzugt endständig daran gebundene, in Wasser dissoziierende ionische Gruppe aus. Die anionische Gruppe kann z. B. eine Sulfat-($-OSO_3^-$), Sulfonat-($-SO_3^-$), Phosphat-($-O-PO_3^{--}$), oder Carboxylat-($-COO^-$)-Gruppe sein, die kationische Gruppe kann z. B. eine quartäre Ammoniumgruppe (z. B. $-N^+(CH_3)_3$) sein, die zwitterionischen Gruppen können z. B. $-N^+-(CH_3)_2-CH_2-COO^-$ oder

$$-N^+(CH_3)_2-CH_2-\underset{OH}{CH}-CH_2-SO_3^-$$ sein.

Geeignete und bevorzugt anionische Tenside sind z. B. Alkylsulfate, Alkylpolyglycolethersulfate, Alkansulfonate, Sulfobernsteinsäuremonoalkylester- und -monopolyethoxyalkylester, Monoalkylphospate, Eiweißfettsäurekondensationsprodukte, Acylisethionate, Acyltauride, Seifen und Acylsarcoside. Geeignete kationische Tenside sind z. B. Alkyl-trimethylammonium-halogenide, Alkyl-dimethyl-benzylammonium-halogenide, Alkyl-pyridinium-halogenide, Alkylimidazolinium-halogenide. Geeignete zwitterionische Tenside sind z. B. N-Alkyl-N,N-dimethylglycin oder N-Acylaminopropyl-N,N-dimethylglycin. Als ionische Tenside können auch Gemische der genannten anionischen oder Gemische aus anionischen oder kationischen und zwitterionischen Tensiden verwendet werden.

Von besonders hohem anwendungstechnischen Interesse ist die Viskositätserhöhung von wässrigen Lösungen der genannten Art mit vergleichsweise geringem Gehalt an ionischen Tensiden. In diesen Fällen verbessert der Zusatz der erfindungsgemäß ausgewählten Verdickungsmittel in synergistischer Weise die Verdickbarkeit mit anorganischen Elektrolytsalzen.

Als anorganische Elektrolytsalze eignen sich alle wasserlöslichen Alkali-, Ammonium- und Erkalkalisalze, z. B. die Fluoride, Chloride, Bromide, Sulfate, Phosphate, Nitrate, soweit sie in einer Menge von wenigstens 1 Gew.-% bei 20 °C in Wasser löslich sind. Bevorzugt werden die Chloride oder Sulfate eines Alkalimetalls, des Ammoniums oder des Magnesiums verwendet. Besonders bevorzugt sind Natriumchlorid und Magnesiumchlorid.

Die erfindungsgemäßen wässrigen Zubereitungen können darüber hinaus weitere Komponenten enthalten, die sie für den jeweiligen Anwendungszweck geeignet machen. In Betracht kommen beispielsweise nichtionogene Tenside, bevorzugt in kleineren Mengen bis höchstens etwa 50 Gew.-% der vorliegenden ionogenen Tenside. In untergeordneten Mengen können schließlich Duftstoffe, Farbstoffe, Trübungs- und Perlglanzmittel, antimikrobielle Stoffe, Konservierungsmittel, hautkosmetische Wirkstoffe, Pflanzenextrakte, Proteinhydrolysate, Puffersubstanzen, Komplexbildner und andere bekannte Hilfs- und Zusatzstoffe enthalten sein, wie sie in Shampoos, Badezusätzen, Duschbadpräparaten, flüssigen Seifen, flüssigen Hautreinigungsmitteln, flüssigen Haarspülmitteln, aber auch in flüssigen Wasch- und Geschirrspülmitteln sowie flüssigen Haushaltsreinigungsmitteln auf Basis von ionogenen Tensiden üblich sind.


## Beispiele

In den nachfolgenden anwendungstechnischen Untersuchungen wurden die sich jeweils einstellenden Viskositätswerte wäßriger Lösungen bestimmt, die der folgenden Grundzusammensetzung entsprechen:

10 Gew.-% (Aktivsubstanz) Tensid auf Ethersulfatbasis

3 Gew.-% Verdickungsmittel

x Gew.-% NaCl

Die Viskositätswerte wurden dabei mit Hilfe des Höppler-Kugelfallviskosimeters bei 20 °C bestimmt und sind in mPas angegeben.

Es wurden dabei in vergleichenden Untersuchungen jeweils für das eingesetzte Verdickungsmittel 3 charakteristische Zahlenwerte bestimmt.

1. Bestimmung der erforderlichen Menge NaCl (x Gew.-%) für die Einstellung des Viskositätswertes von 4 000 mPas

2. Bestimmung des Viskositätswertes (mPas) bei Zusatz von x Gew.-% (gemäß 1.) + 0,5 Gew.-% NaCl

3. Bestimmung des erreichbaren Viskositätsmaximums und Zuordnung der dazu erforderlichen Menge an NaCl in Gew.-%.

In einer ersten vergleichenden Versuchsreihe wurden wäßrige Lösungen des von der Anmelderin unter dem Handelsnamen "Texapon NSO" vertriebenen Produktes (Fettalkohol $C_{12/14}$(70:30)polyglykolether-(2 EO)-sulfat, Na-Salz, 28 %ige Lösung in Wasser) eingesetzt.

In einer zweiten Versuchsreihe wurden entsprechende wäßrige Lösungen auf Basis des von der Anmelderin unter der Handelsbezeichnung "Texapon ASV" vertriebenen Produktes eingesetzt.

Dieses ist eine 30 %ige wäßrige Lösung eines Magnesiumsalzes eines Alkylethersulfats auf Basis einr Mischung von Anlagerungsprodukten von 2 - 10 Mol Ethylenoxid an $C_{12}$-$C_{18}$-Fettalkohole.

Zum Vergleich ist jeweils die entsprechende Verdickungswirkung eines Kokosfettsäurediethanolamids (Handelsprodukt der Anmelderin "Comperlan KD") angegeben.

| Texapon NSO | | | | |
|---|---|---|---|---|
| Produkt | X % NaCl für 4000 mPas | Viskosität (mPas) bei X + 0,5 % NaCl | Viskositätsmaximum mPas | % NaCl |
| Comperlan KD | 1,5 | 16 000 | 30 000 | 3,5 |
| HD-Ocenol 50/55 + 2 EO | 1,5 | 38 000 | 38 000 | 2,0 |
| HD-Ocenol 50/55 + 3 EO | 2,0 | 36 000 | 37 000 | 3,0 |
| HD-Ocenol 50/55 + 4 EO | 2,5 | 21 000 | 33 000 | 3,5 |
| HD-Ocenol 50/55 + 5 EO | 2,5 | 12 000 | 20 000 | 4,0 |
| HD-Ocenol 80/85 + 2 EO | 1,0 | 10 000 | 10 000 | 1,5 |
| HD-Ocenol 80/85 + 3 EO | 2,0 | 39 000 | 39 000 | 2,5 |
| HD-Ocenol 80/85 + 4 EO | 2,0 | 11 000 | 32 000 | 3,5 |
| HD-Ocenol 80/85 + 5 EO | 2,5 | 10 000 | 19 000 | 4,0 |
| Rübocenol dest. + 3 EO | 2,0 | 38 000 | 38 000 | 2,5 |
| HD-Ocenol 50/55 + 2 EO + 1 PO | 1,5 | 18 000 | 36 000 | 2,5 |
| HD-Ocenol 50/55 + 3 EO + 1 PO | 3,0 | 9 000 | 12 000 | 4,5 |
| Texapon ASV | | | | |
| Comperlan KD | 2,5 | 6 500 | 8 000 | 4 |
| HD-Ocenol 50/55 + 3 EO | 2,0 | 9 000 | 15 000 | 3 |
| HD-Ocenol 50/55 + 4 EO | 4,0 | 9 000 | 21 000 | 5,5 |
| HD-Ocenol 50/55 + 5 EO | 5,5 | 7 000 | 9 000 | 7,0 |

**Ansprüche**

1. Verwendung von Polyethoxylaten ein- und/oder mehrfach ungesättigter Fettalkohole mit 12 bis 22 C-Atomen eines mittleren Ethoxylierungsgrades von etwa 2 bis 5, die endständig auch eine bis maximal zwei Propylenoxidgruppen anstelle der entsprechenden Zahl an Ethylenoxidgruppen ankondensiert enthalten können, gegebenenfalls in Abmischung mit Polyethoxylaten gesättigter Fettalkohole als N-freies Verdickungsmittel für wäßrige Tensidlösungen, die auch gelöste anorganische Elektrolytsalze mit Verdickungswirkung enthalten können.

2. Ausführungsform nach Anspruch 1, dadurch gekennzeichnet, daß ungesättigte Fettalkohol-Polyethoxylate eines mittleren Ethoxylierungsgrades von etwa 2 bis 4,5 und insbesondere des Bereiches von etwa 3 bis 4 zum Einsatz kommen.

3. Ausführungsform nach den Ansprüchen 1 und 2, dadurch gekennzeichnet, daß Fettalkohol-Polyethoxylatgemische mit einem überwiegenden Anteil der Kettenlänge $C_{16-20}$, insbesondere $C_{16-18}$ zum Einsatz kommen.

4. Ausführungsform nach den Ansprüchen 1 bis 3, dadurch gekennzeichnet, daß Fettalkohol-Polyethoxylate mit 1 bis 3 ethylenischen Doppelbindungen zum Einsatz kommen.

5. Ausführungsform nach den Ansprüchen 1 bis 4, dadurch gekennzeichnet, daß Gemische von gesättigten und ungesättigten Fettalkohol-Polyethoxylaten verwendet werden, die wenigstens etwa zur Hälfte, bevorzugt zu etwa 55 bis 90 Gew.-%, ungesättigte Fettalkohol-Polyethoxylate enthalten.

6. Ausführungsform nach den Ansprüchen 1 bis 5, dadurch gekennzeichnet, daß die im Gemisch vorliegenden gesättigten Fettalkohol-Polyethoxylate im Bereich der C-Kettenlänge und des Ethoxylierungsgrades den eingesetzten ungesättigten Fettalkohol-Polyethoxylaten etwa entsprechen.

7. Ausführungsform nach den Ansprüchen 1 bis 6, dadurch gekennzeichnet, daß Fettalkoholgemische natürlichen Ursprungs eingesetzt werden.

8. Ausführungsform nach den Ansprüchen 1 bis 7, dadurch gekennzeichnet, daß die ungesättigten Fettalkohol-Polyethoxylate in Aniontenside enthaltenden wäßrigen Wirkstoffmischungen, insbesondere in kosmetischen Präparaten zum Einsatz kommen, die bevorzugt zusätzlich geringe Mengen gelöster anorganischer Elektrolytsalze wie Natriumchlorid enthalten.

9. Verdickte wäßrige Zubereitungen ionischer Tenside, insbesondere für den Einsatz auf dem Gebiet kosmetischer Präparate, enthaltend etwa 5 bis 20 Gew.-% ionischer Tenside zusammen mit etwa 1 bis 5 Gew.-% wasserlöslicher Verdikkungsmittel und 0 bis 10 Gew.-% wasserlöslicher anorganischer Elektrolytsalze sowie gewünschtenfalls übliche Zusatzstoffe kosmetischer Zubereitungen in untergeordneten Mengen, dadurch gekennzeichnet, daß sie als N-freies Verdickungsmittel Polyethoxylate ein- und/oder mehrfach ungesättigter Fettalkohole mit 12 bis 22 C-Atomen eines mittleren Polyethoxylierungsgrades von etwa 2 bis 5, bevorzugt von etwa 3 bis 4, die endständig auch eine bis maximal zwei Propylenoxidgruppen anstelle der entsprechenden Zahl an Ethylenoxidgruppen ankondensiert enthalten können, gegebenenfalls in Abmischung mit Polyethoxylaten gesättigter Fettalkohole des bevorzugt gleichen Bereiches der C-Kettenlänge und des Polyethoxylierungsgrades enthalten.